**Europäisches Patentamt**

**European Patent Office**

**Office européen des brevets**

(19)

(11) **EP 1 571 451 A1**

(12) **EUROPEAN PATENT APPLICATION**

(43) Date of publication:
**07.09.2005 Bulletin 2005/36**

(51) Int Cl.7: **G01N 33/76**

(21) Application number: **04251289.7**

(22) Date of filing: **05.03.2004**

| | |
|---|---|
| (84) Designated Contracting States:<br>**AT BE BG CH CY CZ DE DK EE ES FI FR GB GR HU IE IT LI LU MC NL PL PT RO SE SI SK TR**<br>Designated Extension States:<br>**AL LT LV MK**<br><br>(71) Applicant: **Inverness Medical Switzerland GmbH 6300 Zug (CH)**<br><br>(72) Inventor: **The designation of the inventor has not yet been filed** | (74) Representative: **Lipscombe, Martin John et al Keith W Nash & Co, 90-92 Regent Street Cambridge CB2 1DP (GB)**<br><br><u>Remarks:</u><br>Claim 20 is deemed to be abandoned due to non-payment of the claims fee (Rule 31 (2) EPC). |

(54) **Method and apparatus for estimating due date**

(57)    Disclosed is a method of determining the estimated due date for a pregnant human female subject, the method comprising: testing a sample of a body fluid from the subject so as to obtain data on the concentration of human chorionic gonadotrophin (hCG) in the sample; and applying a suitable algorithm to the hCG concentration data so as to derive an estimated due date.

Fig. 2

EP 1 571 451 A1

**Description**

**Field of the Invention**

The present invention relates to a novel method for determining the estimated due date (EDD) for the birth of a mammalian subject, and apparatus for use in the method. The invention also provides a method and apparatus for determining the probable date of conception.

**Background of the Invention**

The estimated due date (EDD) is the date around which birth can normally be expected. In practice, for humans, as little as 5% of births occur actually on the EDD, but around 90 % occur within the interval of ten days on either side of the EDD. The EDD is very important as many factors, both clinical and non-clinical, depend on it (e.g. arrangement of appointments with obstetricians, decisions to induce labour etc).

At present, two methods of determining the EDD are conventionally employed. The simplest is the "LMP" (or "last menstrual period") method. This requires knowledge of the date of the woman's last period. In the LMP method the EDD may be determined by taking the first day of bleeding in the last period and either (a) adding 7 days and subtracting 3 calendar months (and advancing the year) or (b) adding 280 days (10 lunar months of exactly 28 days). The result of either calculation is essentially similar although the precise result may vary by a day or two because calendar months have different numbers of days.

The LMP method of EDD determination has the advantage of technological simplicity. However, it may not always be reliable (e.g. where a woman cannot remember accurately the date of her most recent period, or experiences highly irregular cycles).

As an alternative, in circumstances where the LMP method may not be appropriate, or as confirmation of an EDD determined by the LMP method, an ultrasound scan may be performed. The size of the foetus can be used to determine the EDD. However, the accuracy of EDDs determined by ultrasound examination is significantly reduced if the scan is performed after the 13th week of pregnancy. Moreover, EDD determination by ultrasound examination requires a skilled practitioner and the use of expensive and cumbersome equipment. It would be advantageous to provide an accurate method of EDD determination which can be performed simply.

It has not, to the knowledge of the inventor, hitherto been suggested that the EDD could be determined on the basis of hormonal measurements.

**Summary of the Invention**

In a first aspect the invention provides a method of determining the estimated due date for a pregnant human female subject, the method comprising: testing a sample of a body fluid from the subject so as to obtain data on the concentration of human chorionic gonadotrophin (hCG) in the sample; and applying a suitable algorithm to the hCG concentration data so as to derive an estimated due date.

In a second aspect the invention provides a method of determining the estimated date of implantation of an embryo, the method comprising: testing a sample of a body fluid from a pregnant human female subject so as to obtain data on the concentration of hCG in the sample; and applying a suitable algorithm to the hCG concentration data so as to derive an estimated date of implantation.

The invention also provides, in a third aspect, a method of determining the estimated date of conception of a human foetus, the method comprising: testing a sample of a body fluid from a pregnant human female subject so as to obtain data on the concentration of hCG in the sample; and applying a suitable algorithm to the hCG concentration data so as to derive an estimated date of conception. Typically the method of the third aspect of the invention is most conveniently performed by employing the method of the second aspect of the invention (i.e. estimating the date of implantation and using that information to estimate the date of conception).

Conveniently hCG concentration data obtained for use in the method of the first aspect of the invention can be used in a method in accordance with the second aspect of the invention and *vice versa*. Accordingly, hCG concentration data from the same sample or set of samples could be used to determine both an EDD and an estimated date of conception.

The sample of body fluid could be any body fluid in which representative levels of hCG are present (e.g. blood, plasma, serum). A preferred body fluid is urine. Methods of determining the concentration of hCG in urine are well known to those skilled in the art.

The methods of the present invention involve determination of the concentration of hCG in a sample of body fluid. The hCG concentration may be determined in a relative manner (e.g. by comparison with a reference or threshold value) but it is much more preferred that the concentration of hCG be determined in an absolute manner (e.g. by measuring the number of mIU of hCG per ml of fluid sample).

Known methods of assaying hCG in samples of body fluid include immunological techniques (i.e. the use of an immunoglobulin, especially a monoclonal antibody, having specific binding for hCG), and such techniques may advantageously be used in the present invention. In particular it is preferred to use an assay technique which can be performed by the woman herself at home, or performed simply by a general practitioner, without resort to laboratory equipment or analysis. In this regard lateral flow assay devices are especially useful, being cheap and simple to use.

Many lateral flow assay devices are known which are suitable for measurement of hCG in urine, as these constitute the basis of most home pregnancy test kits. Examples of such devices are disclosed in EP 0291194.

In general, however, these devices are not ideally suited for use in the method of the present invention, as they provide only a qualitative assay result (i.e. "pregnant" or "not pregnant"), and a quantitative result is much preferred in the present invention. Although it has been known for decades that hCG concentrations rise steeply early in pregnancy (and therefore act as a reliable diagnostic indicator of pregnancy) it has never been proposed that hCG concentration could be used to calculate an estimated date of conception and/or an estimated due date.

Typically home pregnancy test kits involve the use of a dipstick-type lateral flow device impregnated with a labelled hCG-specific immunological reagent which is caused, in the presence of an hCG-containing sample, to accumulate in a test result zone. The label is typically a direct label, such as a coloured bead, or colloidal gold. Visual inspection of the device by the user is necessary, and the user must interpret the assay result based on the amount of label accumulated in the test zone.

One type of hCG assay device is provided with a corresponding reader (the ClearBlue Easy Digital Pregnancy Test device, available from Unipath Limited, Bedford, UK) which includes optical measurement means which can detect and measure the amount of label accumulated. The assay reading device has an LCD window in which to display the assay result. Such an assay device/assay result reader combination could, suitably programmed, be used in the method of the first and/or second and/or third aspects of the invention.

The methods of the invention are such that the EDD and/or estimated date of conception can be calculated from a measurement of hCG concentration obtained from a single sample of body fluid obtained on a single day. If desired, however, hCG concentration may be determined in samples obtained from the subject on two or more days. The inventor has found that determining the hCG concentration in samples obtained on two or three days can, to some extent, enhance the accuracy of the prediction, but that determining hCG concentration in samples obtained on four days provides a more marked improvement in accuracy. In embodiments in which samples of body fluid are obtained on a plurality of days, it is desirable that the sample is taken at about the same time of day (e.g. +/- 1 hour) on each occasion, to reduce variation in sample composition. Where the body fluid sampled is urine, for instance, it is advantageous to perform the hCG assay each day on early morning urine samples, so as to reduce variability due to differences in volume of urine output. If a plurality of samples (obtained on different days) is tested, the samples may be taken on successive days or, in the alternative, a hiatus of one or more days may occur between sampling days, provided that the last day of sampling is within the overall test window.

The hormone concentration is used to estimate the date on which the embryo has been implanted in the uterus. The estimated due date and estimated date of conception may be calculated from the estimated implantation date. The implantation date is estimated by consideration of the hCG levels in conjunction with an empirically determined equation to determine the day on which the hormone concentration started to rise. This day is taken as the estimated day of implantation.

Especially surprising is the finding that one hCG concentration data point <u>can</u> be sufficient to determine date of implantation with reasonable accuracy, especially if the sample is taken early during pregnancy (up to about 15 days post-implantation).

The inventor has found, from statistical analysis, that two sample points can improve accuracy (e.g. in reducing calibration errors in the assay system), especially if the sample points are separated by a hiatus of at least 48 hours. Advantageously, if just two sample points are employed, samples are taken at least 5 days apart, conveniently about 6 or 7 days apart (e.g. one sample at about 15 days post-implantation and another sample at about 22 days post-implantation).

For maximum accuracy it is generally necessary to take at least three, or more preferably at least four, samples. The samples may be taken on successive days but it is preferred that there is a temporal separation of at least about 48 hours between the taking of at least two of the samples. The samples may be taken at either regular or irregular intervals, but each should preferably be taken at about the same time of day. For example, samples might be taken at days 10, 15, 20 and 25 post-implantation, or at days 15, 17, 24 and 26 post-implantation. It should be borne in mind, however, that maximum accuracy in determination of EDD and/or estimated date of conception is not always necessary and for many purposes, calculations based on just one or two data points may be sufficient.

As mentioned above, immunological techniques for determining the concentration of hCG are especially preferred. The properties of immunological reagents are not universal e.g. different hCG-specific antibodies will have different binding affinities. Accordingly, it is not appropriate to define the present invention by reference to any particular algorithm, but those skilled in the art, with the benefit of the present disclosure, will readily be able to ascertain an appropriate algorithm for a particular assay system of interest depending, for example, on the antibody or other reagent used, the label, the detection/measurement system etc.

The inventor has found that there is an exponential rise in the level of hCG early in pregnancy, in both serum and urine samples. A study of the hCG concentration in urine from a large sample of pregnant women showed that whilst the rate of increase of hCG concentration does vary from individual to individual (as might be anticipated), somewhat surprisingly, the rate of increase of hCG concentration was generally consistent across the sample. The inventor realised that this meant that the hCG concentration could be used to extrapolate "backwards" to provide an estimated date of implantation, which could in turn allow determination of an estimated due date and/or an estimated date of conception (conception generally takes place 7 days, +/- 2 days, prior to implantation; and the EDD date can be determined as date of implantation plus 37 weeks).

The exponential increase in hCG concentration following implantation continues for about a further four or five weeks, after which time the hCG concentration tends to plateau, so that hCG measurements made outside this time interval cannot be used to determine accurately the estimated date of implantation.

Accordingly, it is a desirable feature of the methods of the invention that the one or (preferably) more tests of hCG concentration should be made within about 8 weeks, more preferably within 7 weeks, and most preferably within 6 weeks, of the woman's last menstrual period although, unlike the LMP method of EDD determination, there is no requirement for the woman to recall the precise date of her last period.

The method of the present invention should provide an EDD which is more accurate than that provided by the LMP method, as the present invention is based on physiological data. Unlike the ultrasound scan method, the present invention can be performed very easily, does not require the use of expensive equipment, does not require a high degree of skill and training, and can be performed by the pregnant woman herself or by a nurse or general practitioner without the need for an experienced ultrasonographer.

The present inventor has also appreciated the desirability for the method of the invention to be able to measure hCG concentration accurately across a wide range of values, since the hCG concentration increases so rapidly over a short period of time, from 0mIU/ml at the time of implantation to about 1,000 mIU/ml (in urine) by about two weeks after implantation. If necessary, hCG tests conducted shortly after implantation could be performed using one particular measurement technique, whilst hCG tests conducted later could be performed using a different measurement technique. It is preferred however to employ a single technique which is suitable for hCG measurements made at any time during the relevant test window of up to 6-8 weeks following the last menstrual period. Techniques for measurement of hCG concentration which could be employed include HPLC, Western blotting, SDS-PAGE, enzyme or fluorescent immunoassay, but, as mentioned previously, preferred measurement techniques comprise the use of an immunochromatographic lateral flow type device, which is cheap yet sensitive and easy to use.

In a further aspect, the invention provides apparatus for determining the EDD and/or estimated date of conception. The apparatus is typically suitable and adapted for use in the method(s) defined previously. More specifically, the present invention provides apparatus for use in determining the EDD and/or estimated date of implantation and/or date of conception for a human foetus present in a pregnant woman, the apparatus comprising data processing means to process the hCG concentration data according to a suitable algorithm programmed into the apparatus so as to determine an estimated date of implantation and/or an estimated due date and/or an estimated date of conception. The hCG concentration data may be provided to the apparatus e.g. by inputting data from a user interface, such as a keyboard. Alternatively, and preferably, the apparatus may comprise means for measuring the concentration of hCG in a sample of body fluid obtained from the woman, so as to provide hCG concentration data, which can then be directly processed by the data processing means, without the data needing to be input by a user.

The apparatus preferably additionally comprises display means to display the determined EDD and/or estimated date of implantation and/or estimated date of conception. The display means may be an electronic alphanumeric display and may comprise, for example, a liquid crystal display. Alternatively, and less preferably, the apparatus may comprise a data export means, for exporting the determined EDD and/or date of conception data to some other piece of equipment, such as a personal computer provided with a separate display means.

In preferred embodiments, apparatus in accordance with the invention may take the form of a kit comprising an electronic assay result reading device (similar to the "ClearBlue Easy Digital" pregnancy test device, available from Unipath Limited, Bedford, UK) for use with disposable lateral flow assay test sticks. The test sticks themselves will be contacted with a urine sample from a woman and then inserted into the electronic result reading device which, typically, will use an optical system to measure the amount of label accumulated in the test zone of the test stick (e.g. a transmission or reflectance reading to measure the amount of light respectively transmitted through, or reflected from, the test zone which in turn depends on the amount of label deposited in the test zone). The reading device will comprise data processing means which, from the measurements, will determine the concentration of hCG in the sample and process the hCG concentration data according to an algorithm programmed into the device, thereby to arrive at an EDD and/or an estimated date of conception (conveniently by determining, as an intermediate step, an estimated date of implantation).

Advantageously the data processing means will comprise a microprocessor. If the device is to be used in a manner in which hCG tests are conducted on a plurality of days, it will be desirable for the reader to comprise some sort of calendar means (preferably in electronic form) and a data storage device to store the results of previous hCG tests. The reader will preferably also comprise an integral power source, such as button-type lithium cell or other battery, or possibly a solar powered device such as a photovoltaic cell.

Test sticks for use with assay result reader may be generally conventional. However, as mentioned previously, the hCG testing regime is preferably able to provide accurate results over a wide range of different hCG concentrations. Conventional hCG-specific test sticks are not normally configured to provide for this possibility since, in a pregnancy test, a qualitative result is all that is required.

One solution to this problem would be to provide a number of different test sticks say, for example, a "low", "medium" and "high" stick; the "low" stick being for use in tests conducted shortly after implantation and for use in an hCG concentration range of 10-100 mIU/ml; the "medium" stick being for use in an hCG concentration range of 100-1,000 mIU/m and the "high" stick, for use near the end of the testing window, at an hCG concentration range of 1,000-10,000+ mIU/ml.

An alternative, and preferable, solution is to provide a single test stick which is modified so as to be able to provide an accurate result over a wide range of hCG concentration values. One way in which an otherwise conventional test stick might be modified to extend the concentration range over which it can provide an accurate quantitative result is to provide a plurality of test zones, e.g. a "low", "medium" and "high" which correspond to the concentration ranges identified above.

By way of explanation, a single test stick could be provided with an immobilised capture reagent deposited in three discrete test zones: when the hCG concentration is low (early after implementation), substantially all of the hCG will be captured (directly or indirectly) at the "low" test zone furthest upstream. Later samples, containing a higher concentration of hCG, will tend to saturate the "low" test zone, so that significant amounts of hCG will be captured at the "medium" test zone. Later still in the testing window, when samples contain an even higher concentration of hCG, the "medium" test zone will also become saturated and significant amounts of hCG will be captured at the "high" test zone. The reading device will be programmed to distinguish between different zones, e.g. on a positional/spatial basis, so as to determine an accurate result for hCG concentration.

Although the above description refers to the use of three test zones, it should equally be possible to employ a test stick containing, say, two or four hCG concentration test zones, and indeed a two test-zone stick may be preferred as being simpler and cheaper to make and use.

In some embodiments, the method/apparatus of the invention may also comprise measurement of LH in samples of body fluid (preferably urinary LH). Measurement of LH on a frequent basis would allow detection of the LH "surge" which occurs immediately prior to ovulation. Knowledge of the occurrence of ovulation would enable the user to estimate when implantation might take place (approximately 7 days later) and thus when to start testing for hCG. Again, methods of testing urinary LH concentration are well known to those skilled in the art, and convenient methods include the use of lateral flow immunochromatographic devices (e.g. as disclosed in EP 0291194). Thus, in one embodiment, the invention provides a kit comprising: one or more test sticks for measurement of hCG in a sample of body fluid; one or more test sticks for measurement of LH in a sample of body fluid; and an assay result reading device equipped with a suitably programmed data processing means and a display means; the reading device being capable of detecting an LH surge in the LH test results obtained from the sample/s of body fluid and calculating therefrom an hCG test commencement day on which to start testing samples for hCG concentration and indicating said hCG test commencement day to the user via the display means; and wherein said reading device is also programmed to determine and display an EDD and/or estimated date of conception derived from hCG concentration data.

The kit may be provided with two types of test stick: one type for testing LH; and a second type for testing hCG. Alternatively the kit may be provided with a single type of test stick which can be used to determine both LH and hCG concentration (e.g. from a single urine sample applied to the stick).

The method and apparatus of the present invention can be used for a number of purposes. For example, anxious expectant mothers can be reassured early in pregnancy that the pregnancy is proceeding normally, by measurement of hCG and calculation of the EDD. This applies especially to women experiencing their first pregnancy or who have experienced repeated miscarriage. In some instances, determining an estimated date of conception may help to identify the likely father. In addition, unusual hCG concentration data may be used to obtain an early indication of possible problems (e.g. ectopic pregnancy, miscarriage) or of multiple conceptions.

For the avoidance of doubt it is hereby explicitly stated that any feature of the invention described as "preferred", "advantageous", "desirable" or described in similar such terms may be employed in any embodiment of the invention in isolation or in combination with any other feature or features so described, insofar as the context permits.

The invention will now be further described by way of illustrative example, and with reference to the accompanying drawings, in which:

Figures 1 and 2 are graphs showing concentration of hCG in urine (in mIU/ml) against time (days post implantation);

Figure 3 shows a perspective view of an electronic assay result reading device suitable for use in performing the methods of the invention.

**Examples**

Example 1

As part of a larger study into the changes in concentration of various hormones during the female reproductive cycle, samples were gathered from a group of women volunteers. Early morning urine samples were collected on a daily basis, and these were analysed, using standard immunological assay techniques, to obtain values for the concentration of a number of analytes, including urinary hCG, LH, FSH etc.

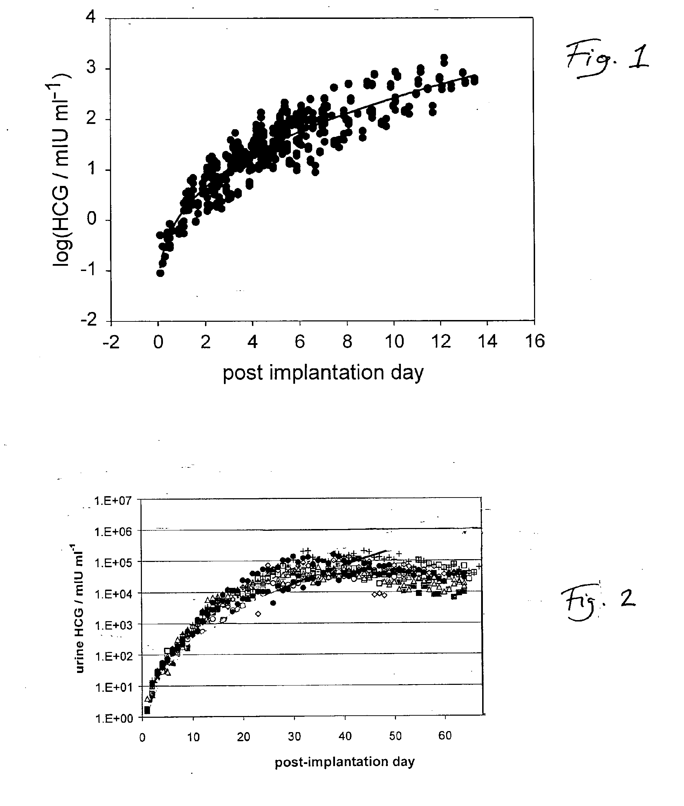

Subsequent analysis of the data showed that, in those women who became pregnant during the course of the study, the urinary concentration of hCG increased rapidly, as expected. Surprisingly, however, the inventor discovered that the graphs of hormone concentration vs time could be shifted on the time axis so as to almost superimpose. Namely, the rate of hCG increase between different individuals did not alter greatly and the difference between different individuals being to a significant extent due to the differences in the time at which the hCG concentration started to rise. The time at which this occurs is taken as the estimated time at which the embryo was implanted in the uterus. Typical results are shown in Figure 1, which is a graph of the log of hCG concentration (in mIU/ml) against time (days post implantation). It can be seen that the individual data points cluster close to an empirically derived regression curve.

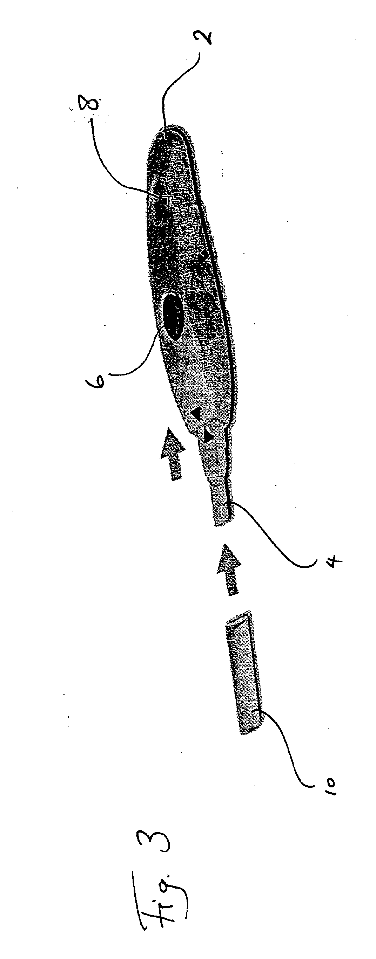

Figure 2 shows similar data, plotted as log of urinary hCG concentration (in mIU/ml) against time (days post-implantation), but continued over a longer period at the start of the pregnancy. This figure shows that after about 25 days post-implantation, the rate of increase of hCG concentration substantially decreases from the logarithmic rate of increase exhibited earlier, and by about 35 days post-implantation the concentration of hCG reaches a plateau, and the method of the invention can no longer be employed with confidence, since it will not be possible to determine accurately the date of implantation and hence the EDD or estimated date of conception.

Example 2

From the data, the inventor was able to derive a number of algorithms which could be used to estimate the day of implantation based on measured hCG concentration values. Using the data illustrated in Figure 1, the following equation was derived:

$$\text{Log (hCG mIU/ml)} = a \left([t - t_o] \text{ days}\right)^{0.33} - 1.86 \qquad \text{(equation 1)}$$

where $t_o$ is the implantation date and a is a coefficient (in this instance 1.99).

From the data illustrated in Figure 2, which were obtained using a slightly different hCG assay technique, the value of a was found to be 2.15.

From this, it follows that the equation to calculate the time (in days) which has elapsed since implantation, given a single measured value for hCG concentration is

$$t - t_o \text{ (days)} \approx \frac{(\log (\text{hCG mIml}) + 1.86)^3}{a} \qquad \text{(equation 2)}$$

From the calculated day of implantation, the EDD is determined by adding 37 weeks. The date of conception is estimated by substracting 7 days ($^+$/- 2 days) from the date of implantation.

Example 3

A cause of uncertainty in the calculation of the date of implantation is that the value of the parameter 'a' varies from person to person (although by less than might have been predicted prior to the present disclosure). Another cause of uncertainty is that the urinary hCG concentration fluctuates randomly about the mean value throughout any given day.

The inventor investigated whether these causes of uncertainty can be reduced by using a number of hCG concentration determinations (e.g. two measurements, taken 7 days apart).

Attempts to estimate parameters *a* and $t_0$ by direct solution of equation (2) using two different values of log (HCG) obtained on two different days failed, because cubing the value (log(HCG) -1.86) amplified the errors. Small errors in the estimation of *a*, caused by small fluctuations in log(HCG), caused large errors in the estimate of $t_0$. The following method seemed to work satisfactorily, however.

From equation 1, the slope of the log(hCG) - time curve is

$$\frac{d\left[\log\left(hCG/mIU\,ml^{-1}\right)\right]}{dt} = 0.33a\left(t - t_0\right)^{-0.67}$$

hence

$$t - t_0 = \frac{\left[\log\left(hCG/mIU\,ml^{-1}\right)\right] + 1.86}{3.d\left[\log\left(hCG/mIU\,ml^{-1}\right)\right]\Big/dt}$$

If two values are taken *n* days apart, $t_1$ is the time of the first measurement and $\overline{\left[\log\left(hCG/mIUml^{-1}\right)\right]}$ denotes the mean value of the two measurements, then

$$t_1 - t_0 + n/2 \approx \frac{\overline{\left[\log\left(hCG/mIU\,ml^{-1}\right)\right]} + 1.86}{3.\Delta\left[\log\left(hCG/mIU\,ml^{-1}\right)\right]\Big/n}$$

If three or more measurements are taken then a standard regression method may be applied to equation 2 to obtain t(0) without prior assumption as to the value of "a". If the estimated value of "a" falls outside the expected range, then the algorithm can prompt for further measurements to improve the accuracy of the estimate or can alert for the need to explore potential complications of the pregnancy.

Example 4

Apparatus in accordance with the invention, and suitable for use in accordance with the methods of the invention, takes the form of a kit comprising an electronic, battery powered assay result reading device and a plurality (typically three or four) test sticks for measuring the concentration of hCG in urine samples. The assay result reading device is closely similar to the ClearBlue Easy Digital reading device commercially available from Unipath Limited (Bedford, UK) and is illustrated in Figure 3.

The reading device comprises an opaque housing 2 formed from a synthetic plastics material. At one end of the housing 2 is an aperture into which a test stick 4 can be inserted. Various guidance and positioning means are provided, such that the test stick 4 can be placed into a precise location relative to the reader. The reading device further comprises: an eject button 6, activation of which causes the test stick 4 (when read) to be ejected from the reading device; and an LCD window 8 which can be used to display information and messages. The test stick is provided with a removable cap 10.

Inside the opaque housing 2, and hence not visible in Figure 3, are the optics for performing optical measurements (e.g. transmission or reflectance readings) on the test stick, which measurements are dependent on the amount of label deposited in a test zone or zones on the test stick, which in turn depends on the concentration of analyte present in the sample applied to the test stick.

The reading device further comprises a button-type flat lithium cell, a microprocessor, and a memory device. The microprocessor is programmed so as to calculate the hCG concentration from the optical measurements made, and additionally programmed with a suitable algorithm to use the derived hCG concentration data to determine the date of implantation, and hence the most likely date of conception and the estimated due date, which two pieces of information can be displayed in the liquid crystal display window 8.

**Claims**

1. A method of determining the estimated due date for a pregnant human female subject, the method comprising: testing a sample of a body fluid from the subject so as to obtain data on the concentration of human chorionic gonadotrophin (hCG) in the sample; and applying a suitable algorithm to the hCG concentration data so as to derive an estimated due date.

2. A method of determining the estimated date of conception of a human foetus, the method comprising: testing a sample of a body fluid from a pregnant human female subject so as to obtain data on the concentration of hCG in the sample; and applying a suitable algorithm to the hCG concentration data so as to derive an estimated date of conception.

3. A method of determining the estimated date of implantation of an embryo, the method comprising: testing a sample of a body fluid from a pregnant human female subject so as to obtain data on the concentration of hCG in the sample; and applying a suitable algorithm to the hCG concentration data so as to derive an estimated date of implantation.

4. A method according to claim 1 or 2, comprising the step of estimating the date of implantation of the embryo, and calculating therefrom the due date or estimated date of conception, as appropriate.

5. A method according to any one of the preceding claims, wherein the determination is made on the basis of a single hCG concentration measurement.

6. A method according to any one of claims 1-4, wherein the determination is made on the basis of a plurality of hCG concentration measurements.

7. A method according to any one of the preceding claims wherein the hCG concentration data are obtained from a sample or samples collected in the time interval up to 15 days post-implantation.

8. A method according to any one of claims 1-6, wherein the hCG concentration data are obtained from a sample or samples collected in the time interval up to 8 weeks following the female subject's last menstrual period.

9. A method according to claim 8, wherein the sample or samples are collected in the time interval up to 7 weeks following the female subject's last menstrual period.

10. A method according to claim 8, wherein the sample or samples are collected in the time interval up to 6 weeks following the female subject's last menstrual period.

11. A method according to any one of the preceding claims, wherein the body fluid sampled is urine.

12. A method according to any one of the preceding claims, wherein hCG concentration data are obtained by conducting a lateral flow immunoassay using the sample of body fluid.

13. A method according to any one of the preceding claims, further comprising the step of monitoring the subject to detect ovulation or an event associated with ovulation.

14. A method according to claim 13, wherein the monitoring comprises measurement of LH in a sample of body fluid, preferably urine, periodically collected from the subject.

15. Apparatus for use in a method in accordance with any one of the preceding claims, the apparatus comprising data processing means to process hCG concentration data according to a suitable alogorithm programmed into the apparatus, thereby to determine an estimated date of implantation, and/or an estimated date of conception, and/or an estimated due date.

16. Apparatus according to claim 15, further comprising means for measuring the concentration of hCG in a sample of body fluid obtained from a subject, so as to provide hCG concentration data.

17. Apparatus according to claim 15 or 16, further comprising a display means.

**18.** Apparatus according to any one of claims 15, 16 or 17, further comprising means for measuring the concentration of LH in a sample of body fluid obtained from a subject and, optionally, means for displaying to a user when to conduct an assay to measure hCG concentration.

**19.** Apparatus according to any one of claims 15-18, comprising one or more disposable, lateral flow assay test sticks and an electronic assay result reader.

**20.** Apparatus according to any one of claims 15-19 and substantially as hereinbefore described.

Fig. 1

Fig. 2

Fig. 3

EP 1 571 451 A1

**European Patent Office**

**EUROPEAN SEARCH REPORT**

Application Number

EP 04 25 1289

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 2002/123671 A1 (HAALAND PETER D) 5 September 2002 (2002-09-05) * page 6, paragraph 51 - page 7, paragraph 52 * ----- | 1-6, 15-17,19 | G01N33/76 |
| Y | EP 0 983 748 A (UNIPATH LTD) 8 March 2000 (2000-03-08) * page 1 - page 24 * ----- | 1-19 | |
| Y | WO 99/64860 A (UNIV OXFORD ; QUEEN MARY & WESTFIELD COLLEGE (GB); REDMAN CHRISTOPHER) 16 December 1999 (1999-12-16) * page 1 - page 23 * ----- | 1-19 | |
| A | US 5 928 168 A (LAROS JR RUSSELL K) 27 July 1999 (1999-07-27) Whole document ----- | 1-19 | |
| A | US 5 876 335 A (HANDY JEFFREY D ET AL) 2 March 1999 (1999-03-02) Whole document ----- | 1-19 | TECHNICAL FIELDS SEARCHED (Int.Cl.7) G01N |

~~The present search report has been drawn up for all claims~~

| Place of search | Date of completion of the search | Examiner |
|---|---|---|
| The Hague | 20 July 2004 | Angioni, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent

Office

## CLAIMS INCURRING FEES

The present European patent application comprised at the time of filing more than ten claims.

[X] Only part of the claims have been paid within the prescribed time limit. The present European search report has been drawn up for the first ten claims and for those claims for which claims fees have been paid, namely claim(s):

11-19

[ ] No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

[ ] All further search fees have been paid within the fixed time limit. The present European search report has been drawn up for all claims.

[ ] As all searchable claims could be searched without effort justifying an additional fee, the Search Division did not invite payment of any additional fee.

[ ] Only part of the further search fees have been paid within the fixed time limit. The present European search report has been drawn up for those parts of the European patent application which relate to the inventions in respect of which search fees have been paid, namely claims:

[ ] None of the further search fees have been paid within the fixed time limit. The present European search report has been drawn up for those parts of the European patent application which relate to the invention first mentioned in the claims, namely claims:

**EP 1 571 451 A1**

### ANNEX TO THE EUROPEAN SEARCH REPORT
### ON EUROPEAN PATENT APPLICATION NO.

EP 04 25 1289

This annex lists the patent family members relating to the patent documents cited in the above-mentioned European search report.
The members are as contained in the European Patent Office EDP file on
The European Patent Office is in no way liable for these particulars which are merely given for the purpose of information.

20-07-2004

| Patent document cited in search report | | Publication date | Patent family member(s) | | Publication date |
|---|---|---|---|---|---|
| US 2002123671 | A1 | 05-09-2002 | AU | 8703101 A | 13-03-2002 |
| | | | WO | 0218933 A2 | 07-03-2002 |
| EP 0983748 | A | 08-03-2000 | EP | 0706346 A1 | 17-04-1996 |
| | | | EP | 0983748 A2 | 08-03-2000 |
| | | | GR | 3034296 T3 | 29-12-2000 |
| | | | AT | 193430 T | 15-06-2000 |
| | | | AT | 236575 T | 15-04-2003 |
| | | | AU | 771932 B2 | 08-04-2004 |
| | | | AU | 2306001 A | 03-05-2001 |
| | | | AU | 7227894 A | 24-01-1995 |
| | | | AU | 8001598 A | 01-10-1998 |
| | | | BR | 9406943 A | 06-08-1996 |
| | | | CA | 2166081 A1 | 12-01-1995 |
| | | | CA | 2271506 A1 | 12-01-1995 |
| | | | CN | 1129898 A | 28-08-1996 |
| | | | CZ | 9600005 A3 | 12-06-1996 |
| | | | DE | 69424789 D1 | 06-07-2000 |
| | | | DE | 69424789 T2 | 23-11-2000 |
| | | | DE | 69432491 D1 | 15-05-2003 |
| | | | DE | 69432491 T2 | 08-04-2004 |
| | | | DK | 706346 T3 | 09-10-2000 |
| | | | WO | 9501128 A1 | 12-01-1995 |
| | | | ES | 2148336 T3 | 16-10-2000 |
| | | | ES | 2196699 T3 | 16-12-2003 |
| | | | HU | 74629 A2 | 28-01-1997 |
| | | | JP | 11133031 A | 21-05-1999 |
| | | | JP | 2907553 B2 | 21-06-1999 |
| | | | JP | 8512132 T | 17-12-1996 |
| | | | NZ | 268889 A | 24-10-1997 |
| | | | PL | 312299 A1 | 15-04-1996 |
| | | | PT | 706346 T | 30-11-2000 |
| | | | ZA | 9404677 A | 29-12-1995 |
| WO 9964860 | A | 16-12-1999 | AU | 751415 B2 | 15-08-2002 |
| | | | AU | 4280299 A | 30-12-1999 |
| | | | CA | 2332349 A1 | 16-12-1999 |
| | | | EP | 1084412 A2 | 21-03-2001 |
| | | | WO | 9964860 A2 | 16-12-1999 |
| | | | JP | 2002517755 T | 18-06-2002 |
| | | | US | 6735529 B1 | 11-05-2004 |
| US 5928168 | A | 27-07-1999 | NONE | | |
| US 5876335 | A | 02-03-1999 | NONE | | |

EPO FORM P0459

For more details about this annex : see Official Journal of the European Patent Office, No. 12/82